# EUROPEAN PATENT SPECIFICATION

(11) **EP 3 287 516 B1**
(45) Date of publication and mention of the grant of the patent: **06.05.2026**
(21) Application number: 16764727.0
(22) Date of filing: 03.03.2016
(51) Int. Cl.: C12M 3/00, C12M 1/00, C12M 1/32, C12M 1/26, C12M 1/12, C12M 1/36, C12M 1/34

(54) **PRODUCTION DEVICE FOR CULTURED-CELL PRODUCT**
HERSTELLUNGSVORRICHTUNG FÜR ZELLKULTURPRODUKT
DISPOSITIF DE PRODUCTION POUR PRODUIT DE CELLULES APRÈS CULTURE

(30) Priority: 18.03.2015 JP 2015054654
(43) Date of publication of application: 28.02.2018
(73) Proprietor: Rohto Pharmaceutical Co., Ltd., Osaka-shi Osaka 544-8666 (JP)
(72) Inventor: KOIKE, Tetsuo, Osaka-shi Osaka 544-8666 (JP); TAKIMOTO, Masahiro, Osaka-shi Osaka 544-8666 (JP); YAGI, Yoshiki, Kuse-gun Kyoto 613-0036 (JP)
(74) Representative: DTS Patent- und Rechtsanwälte PartmbB
(86) International application number: PCT/JP2016/056678
(87) International publication number: WO 2016/147897

(56) References cited:
- EP-A1- 1 650 291
- EP-A1- 1 659 169
- EP-A1- 2 363 255
- EP-A1- 3 162 887
- WO-A2-2011/130865
- JP-A- 2006 149 268
- JP-A- 2007 037 422
- JP-A- 2009 291 104
- JP-A- H02 295 418
- KATSUMI NAKAJIMA ET AL.: "Kan Saibo no Jido Baiyo System", JOURNAL OF THE SOCIETY FOR BIOSCIENCE AND BIOENGINEERING, vol. 92, no. 9, 2014, Japan, pages 473 - 478, XP009503803
- KATSUMI NAKAJIMA ET AL.: "Development of the automatic cell processing machine for the adherent cell", INFLAMM. REGEN., vol. 29, no. 2, 2009, pages 131 - 134, XP055095336

## Description

### CROSS-REFERENCE TO RELATED APPLICATION

### FIELD

The present invention relates to a production apparatus of cultured cell products configured to mass-produce cultured cell products formed by subdividing cultured cells into a large number of containers.

### BACKGROUND

In recent years, cell culture is performed using tissues and cells of various sites of human body, fertilized eggs, or the like, and the cultured cells have been put to practical use for regenerative medicine. In the cell culture, it is important to prevent contamination of cells or the like by bacteria or the like during the culture. Therefore, an automatic culture apparatus that enables the culture of cells using a robot within a housing having a configuration that can maintain thereinside in aseptic conditions has been already proposed (for example, Patent Literature 1).

The aforementioned automatic culture apparatus is configured by arranging, within the housing, an incubator configured to house a culture vessel in which the cells are cultured, a refrigerated cabinet configured to store chemicals, a centrifuge, various containers such as a liquid medicine container and a culture vessel necessary for the culture, and one operation robot configured to handle these components.

Currently, regenerative medicine has been in the limelight, and therefore it is desired to culture a large amount of cells that can be used for regenerative medicine or the like so as to mass-produce cultured cell products.

### CITATION LIST

### Patent Literature

Patent Literature 1: JP 2009-291104 A
EP1659169A1 describes a culture treating apparatus and autoculturing apparatus preventing a contamination into culture containers.
EP1650291A1 describes an automatic cell cultivation apparatus utilizing autoclave sterilization, wherein the apparatus includes an operation unit.
WO2011130865A2 describes an automated cell culture arrangement including a tool module configured to act upon or to monitor the contents of a bioreactor and being movable relative to at least one closed cell culture module.
EP2363255A1 describes an isolator for cell culture.
Katsumi Nakajama et al., Journal of the Society for Bioscience and Bioengineering, vol. 92, No. 9, 2014, pages 473-478 describes an automated cell processing machine for use in regenerative medicine applications.

### SUMMARY

### Technical Problem

In the aforementioned configuration of Patent Literature 1, the operation robot merely performs a process to simply culture cells. Therefore, the cultured cells need to be taken out by human hands. Therefore, there is an inconvenience that the mass production is impossible, and the adoption of regenerative medicine is not widespread.

The present invention has been devised in view of the problems described above, and an object thereof is to provide an apparatus to produce cultured cell products which can produce a large amount of cultured cell products by culturing a large amount of cells and can subdivide the large amount of cultured cells.

### Solution to Problem

The present invention relates to an apparatus to produce cultured cell products having the features of claim 1. An apparatus to produce cultured cell products according to the present invention includes: an incubator configured to house cell culture vessels, an isolator capable of maintaining its inside in aseptic conditions and configured to process the cell culture vessels conveyed from the incubator, and a pass box capable of carrying, into the isolator, articles such as a plurality of types of containers including product containers and reagents which are used for subdividing and placing the cells cultured in the cell culture vessels, wherein the isolator includes: an observation section including first robot arms configured to move the cell culture vessels to an observation position so that the degree of growth of the cells in the cell culture vessels taken out of the incubator is checked; a processing section provided continuously with the observation section and including second robot arms configured to perform various processes to transfer the cells as a cell-containing liquid in the cell culture vessels that have a specified number of cells out of the cell culture vessels observed in the observation section into a large number of product containers carried in from the pass box; and an outlet configured to allow the large number of product containers into which the cells have been transferred to be taken out from the isolator to the outside of the apparatus, wherein at least one first robot arm corresponds to the incubator and is configured to handle the culture vessels that are housed in the incubator, at least one second robot arm corresponds to the pass box and is configured to handle the articles and the reagents to be housed or that have been housed in the pass box, at least one second robot arm corresponds to the outlet and is configured to handle the product containers to be moved from the outlet to the outside of the isolator, and a first robot arm can directly deliver the articles to a second robot arm.

Further, in the apparatus to produce cultured cell products according to the present invention, the processing section may include: a first transfer processing unit configured to transfer a cell-containing liquid housed in the cell culture vessels received from at least one first robot arm into a centrifuge tube, using at least one second robot arm; a separation processing unit configured to separate the cells and a liquid portion by subjecting the centrifuge tube to centrifugation using at least one second robot arm; and a second transfer unit configured to at least partially remove the liquid portion separated by the separation processing unit from the centrifuge tube, put a preservative solution into the centrifuge tube, and transfer a specified number of cells within the centrifuge tube into the large number of product containers while maintaining such a state, using at least one second robot arm.

Further, in the apparatus to produce cultured cell products according to the present invention, the processing section may include: a medium-replacing unit configured to replace a culture medium in the cell culture vessels taken out of the incubator using at least one second robot arm, wherein the medium-replacing unit may be configured to open caps provided on the cell culture vessels received from at least one first robot arm to dispose of the culture medium in the cell culture vessels, supply another culture medium into the cell culture vessels, and put the caps on the cell culture vessels to return the cell culture vessels to at least one first robot arm, using at least one second robot arm.

Further, in the apparatus to produce cultured cell products according to the present invention, at least one second robot arm may be conf igured to open and close screw caps provided on the plurality of types of containers.

### BRIEF DESCRIPTION OF DRAWINGS

Fig. 1 is a front view of an apparatus to produce cultured cell products of the present invention.
Fig. 2 is a plan view of the aforementioned production apparatus.
Fig. 3 is a view of the aforementioned production apparatus as seen from the outlet side.
Fig. 4 is an explanatory diagram showing the state immediately before a vessel cap is opened by a robot arm.
Fig. 5 is a flowchart showing a process of thawing frozen cells and seeding the cells.
Fig. 6A is a flowchart showing a process of thawing frozen cells and seeding the cells.
Fig. 6B is a flowchart showing a process of thawing frozen cells and seeding the cells.
Fig. 7 is a flowchart showing a process of thawing frozen cells and seeding the cells.
Fig. 8 is a flowchart of a process of collecting the cultured cells and passaging the cultured cells.
Fig. 9 is a flowchart of a process of collecting the cultured cells and passaging the cultured cells.
Fig. 10 is a flowchart of a process of collecting the cultured cells and passaging the cultured cells.
Fig. 11 is a flowchart of a process of collecting the cultured cells and passaging the cultured cells.
Fig. 12 is a flowchart showing a process of collecting the cultured cells after the passage process and subdividing the cultured cells into products.
Fig. 13 is a flowchart showing a process of collecting the cultured cells after the passage process and subdividing the cultured cells into products.
Fig. 14 is a flowchart showing a process of collecting the cultured cells after the passage process and subdividing the cultured cells into products.
Fig. 15 is a flowchart showing a process of collecting the cultured cells after the passage process and subdividing the cultured cells into products.

### DESCRIPTION OF EMBODIMENTS

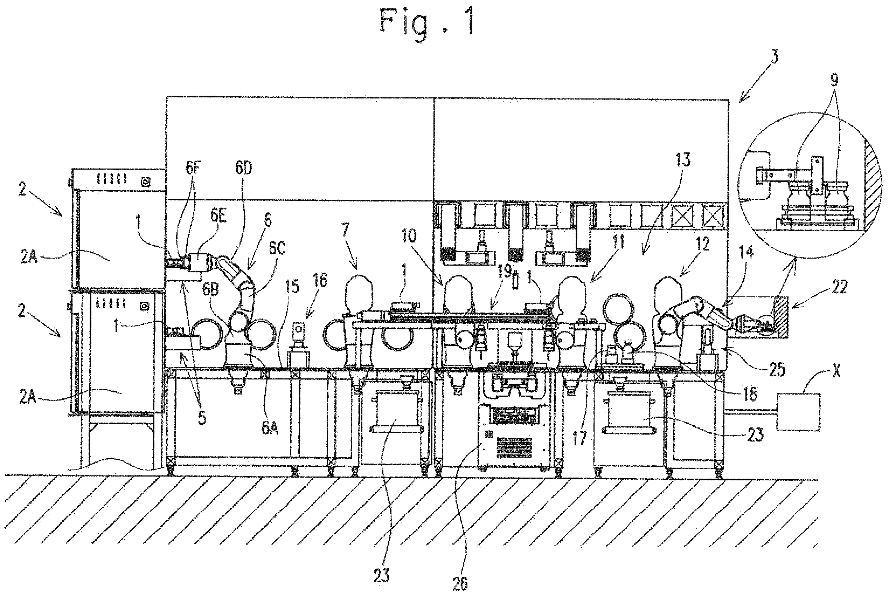

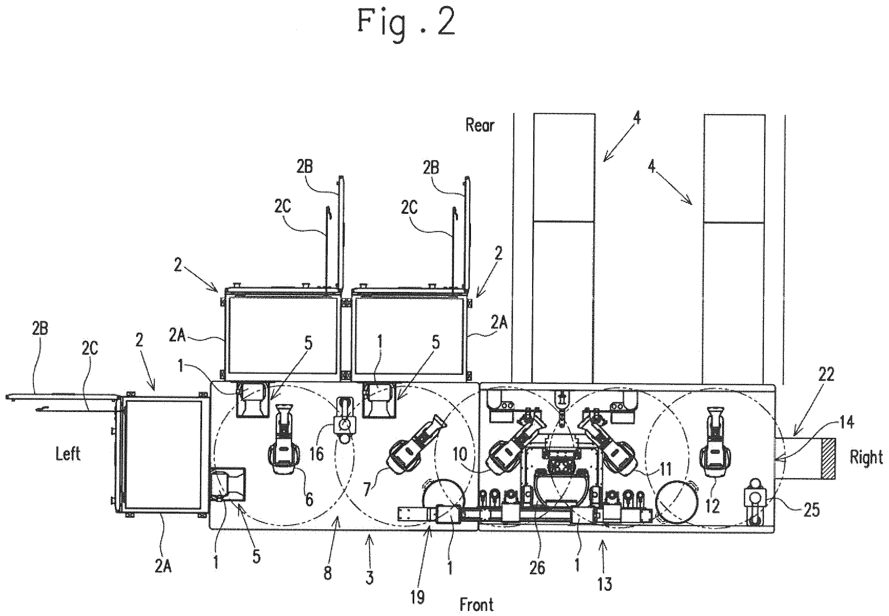

Hereinafter, an apparatus to produce cultured cell products (hereinafter, referred to as production apparatus) according to an embodiment of the present invention, and a method for producing cultured cell products which is implemented using the production apparatus will be described. In the following description on the front, rear, left, and right directions, the left and right directions correspond to the state shown in Fig. 1 and Fig. 2, and the front and rear directions correspond to the state of Fig. 2 where the lower side corresponds to the "front" and the upper side corresponds to the "rear" (the directions are shown also in Fig. 2).

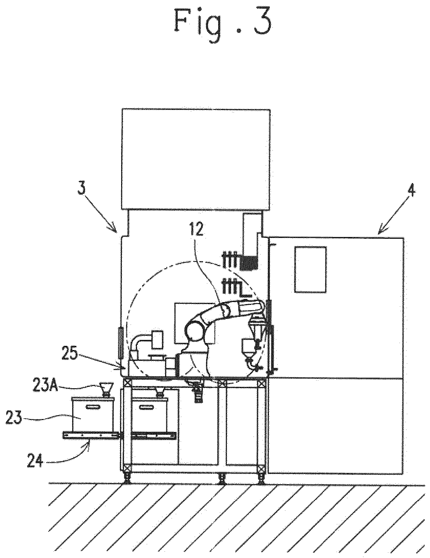

Fig. 1 to Fig. 3 show the production apparatus of this embodiment. The production apparatus includes a plurality of incubators 2 configured to house cell culture vessels (hereinafter, referred to as culture vessels) 1, a horizontally elongated isolator 3 capable of maintaining its inside in aseptic conditions and configured to process the culture vessels 1 conveyed from the incubators 2, and a plurality (two in Fig. 2) of pass boxes 4 capable of carrying, into the isolator 3, articles and reagents which are used for subdividing cells cultured in the culture vessels 1 and placing them. As the culture vessel 1, a HYPERFlask (manufactured by Corning Incorporated in Japan) capable of culturing cells in multilayers, for example, can be used. Each part is controlled by a control device X schematically shown in Fig. 1. The control device X may be provided integrally with the production apparatus or may be a separate body (such as a personal computer) connected to the production apparatus in a wired or wireless manner. Further, it is also possible to provide the control device X integrally with the production apparatus and provide only an operation section of the control device X that is operated by an operator (such as a tablet terminal) as a separate body from the production apparatus.

6 units of the incubators 2 are provided in total in a state of being vertically stacked in two stages as shown in Fig. 1, at three points in total including one point at the left end of the isolator 3 and two points in the left end part behind the isolator 3, as shown in Fig. 2. Two incubators 2 in the upper stage and the lower stage have the same configuration, and each of the incubators 2 is provided with racks (not shown) capable of housing a large number of the culture vessels 1 within a casing 2A. The casing 2A has a box shape with one lateral side opening so that the culture vessels 1 can be taken in and out through the lateral side. Two doors 2B and 2C configured close the opening on the one lateral side of the casing 2A are attached to the casing 2A so as to be freely openable. For example, when the inside door 2C is formed with a transparent material, the number of the culture vessels 1 housed therein and the state of the cell culture can be checked only by opening the outside door 2B while the opening is closed by the inside door 2C. Further, carbon dioxide gas for adjusting the culture atmosphere is configured to be supplied into the incubators 2. Further, the culture vessels 1 housed on the racks inside the incubators 2 are configured to be delivered onto a plurality of mounting tables 5 provided in the isolator 3 by a delivery mechanism, which is not shown. 6 units of the mounting tables 5, which is the same number as the number of the incubators 2, are arranged corresponding to the incubators 2.

As described above, the isolator 3 is horizontally elongated (in this embodiment, it is rectangular in plan view), where one set (two units on the upper and lower sides) of incubators 2 is located on the short side of the isolator 3 (in this embodiment, the left side), and a plurality sets (in this embodiment, two sets (4 units)) of incubators 2 are located on the longitudinal side (in this embodiment, the rear side). This configuration can reduce the size of the production apparatus without reducing the number of cultured cells.

The isolator 3 includes: an observation section 8 including two first robot arms 6 and 7 configured to move the culture vessels 1 to an observation position so that the degree of growth in the culture vessels 1 taken out of the incubators 2 is checked; a processing section 13 provided continuously with the observation section 8 and including three second robot arms 10, 11, and 12 configured to perform various processes to transfer cells in the culture vessels 1 that have a specified number of cells out of the culture vessels 1 observed in the observation section 8 into a large number of product containers 9 (such as vial containers, see the enlarged view of Fig. 1) carried in from the pass boxes 4; and an outlet 14 configured to allow the large number of product containers 9 into which the cells have been transferred to be taken out therethrough. A large number of work gloves (not shown) that allow the operator to perform operations by putting their hands into the isolator 3 are attached onto the front and rear walls of the isolator 3. As shown in Fig. 2, the five robot arms 6, 7, 10, 11, and 12 are aligned in a straight line extending in the left and right directions along the longitudinal direction of the isolator 3.

With reference to the left and right directions, the first robot arm 6 on the left side corresponds to one set of incubators 2 located on the short side of the isolator 3 (in this embodiment, on the left side) and one set of incubators 2 on the left side out of the sets of incubators 2 located on the longitudinal side (in this embodiment, on the rear side), and can handle the culture vessels 1 that are housed in these incubators 2 (the range that can be reached by each robot arm (in plan view) is shown in Fig. 2 with a dashed-double-dotted circle). Further, the first robot arm 7 on the right side corresponds to one set of incubators 2 on the right side out of the sets of incubators 2 located on the longitudinal side of the isolator 3, and can handle the culture vessels 1 housed in the incubators 2.

Further, with reference to the left and right directions, the second robot arm 10 on the left side and the second robot arm 11 in the middle correspond to the pass box 4 on the left side out of the pass boxes 4 located on the longitudinal side of the isolator 3 (in this embodiment, on the rear side), and can handle articles and reagents to be housed (or that have been housed) in the pass box 4.

Further, the second robot arm 12 on the right side corresponds to the pass box 4 on the right side out of the pass boxes 4 located on the longitudinal side of the isolator 3 (in this embodiment, on the rear side) and a box 22 for carrying out the product containers 9, and can handle articles and reagents to be housed (or that have been housed) in the pass box 4 and the product containers 9 to be housed in the box 22.

Further, as seen from the overlapping of the dashed-double-dotted circles shown in Fig. 2, the five robot arms 6, 7, 10, 11, and 12 are arranged in a positional relationship so as to be capable of passing articles to each other.

In this way, the robot arms 6, 7, 10, 11, and 12 are located within the isolator 3, thereby enabling each of the robot arms 6, 7, 10, 11, and 12 to act on the incubators 2, the isolator 3, the pass boxes 4, and the box 22 according to the purpose. This can improve the working efficiency and can contribute to mass production of cultured cell products.

The first robot arms 6 and 7 and the second robot arms 10, 11, and 12 in this embodiment have the same configuration, and therefore only the first robot arm 6 located at the left end will be described. The first robot arm 6 is constituted by articulated robot arm and includes a fixed part 6A fixed to a base member 15 of the isolator 3, a base part 6B that is pivotable about the vertical axis at the distal end part of the fixed part 6A, a first arm 6C that is swingable about the horizontal axis at the distal end part of the base part 6B, a second arm 6D that is swingable about the horizontal axis at the distal end part of the first arm 6C, a third arm 6E that is swingable about the horizontal axis at the distal end part of the second arm 6D, and a pair of grips 6F that are attached to the distal end of the third arm 6E so as to be opposed thereto. The pair of grips 6F are configured to be capable of moving close to and away from each other. The articulated first robot arms 6 and 7 configured as above hold the culture vessels 1 delivered from the incubators 2 using the pair of grips 6F (see Fig. 1) and move them to a microscope 16 at the observation position. The second robot arms 10, 11, and 12 are configured to hold a centrifuge tube 17 and a preparation tank 18 shown in Fig. 1 in addition to the culture vessels 1 so as to be capable of performing various processes.

The microscope 16 at the observation position is arranged between the two first robot arms 6 and 7. By arranging the microscope 16 as above, it is possible to move the culture vessels 1 to the microscope 16 using the first robot arm 6 on the left side so as to observe the cells, and as a result of the observation, it is possible to hold the culture vessels 1 that have been determined to have a specified number of cells so as to rapidly move them to the processing section 13 side, using the first robot arm 7 on the right side. In short, the first robot arm 6 on the left side mainly performs the operation to move the culture vessels 1 to the microscope 16, and the first robot arm 7 on the right side performs the operation to move the culture vessels 1 that have been determined to have a specified number of cells toward the processing section 13 side, so that the operation speed can be accelerated. The determination to have a specified number of cells may be made by counting the number of cells by visual inspection of the operator (human) of the production apparatus or may be automatically made by the control device X based on the number of cells calculated by analyzing an image captured by a camera that is provided in the isolator 3 so as to calculate the number of cells automatically. The culture vessels 1 that are delivered from the incubator 2 located opposed to the first robot arm 7 on the right side are held by the first robot arm 7 on the right side to be moved to the microscope 16. Further, a microscope 25 for observing the cells is provided also in the processing section 13. The object observed by the microscope 25 such as a hemocytometer is held by the second robot arm 12 on the right end to be moved.

Further, the culture vessels 1 after the observation are conveyed not only by being directly passed from the first robot arm 7 on the right side to the second robot arms 10 arranged at the left end of the processing section 13. For example, in the case where the second robot arm 10 is in an operation, the culture vessels 1 after the observation are conveyed by a conveying apparatus 19 to a position where the second robot arm 10 at the left end of the processing section 13 or the second robot arm 11 arranged at horizontal center of the processing section 13 can grip them. The conveying apparatus 19 is provided along the front sidewall of the isolator 3 and is set to a length that allows the conveying apparatus 19 to convey them from the right end part of the observation section 8 of the isolator 3 to the horizontal center of the processing section 13. Accordingly, when the first robot arm 7 on the right side passes the culture vessels 1 after the observation to the conveyance starting end part of the conveying apparatus 19, the conveying apparatus 19 conveys the culture vessels 1 to the position where one of the two second robot arms 10 and 11 can grip them.

The conveying apparatus 19 is provided corresponding to at least one robot arm (in this embodiment, the first robot arm 7) located in the observation section 8 and a plurality of robot arms (in this embodiment, the two second robot arms 10 and 11) located in the processing section 13. The first robot arm 7 can directly deliver the articles to the second robot arm 10. The conveying apparatus 19 can deliver the articles to the first robot arm 7 and the third robot arm 11 between which direct delivery of the articles is impossible. Therefore, even in the case where the articles cannot be delivered from the first robot arm 7 to the third robot arm 11 via the second robot arm 10 due to the second robot arm 10 being in operation, the articles can be delivered from the first robot arm 7 to the third robot arm 11 via the conveying apparatus 19. Therefore, the articles can be conveyed in parallel (via a plurality of routes) within the isolator 3. Accordingly, the working efficiency within the isolator 3 can be improved, and thus the productivity can be improved.

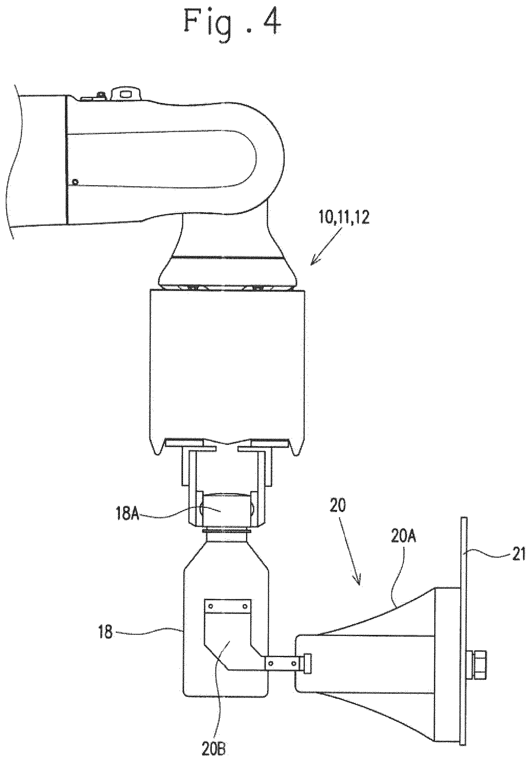

In the processing section 13, three units of the second robot arms 10, 11, and 12 are arranged at equal intervals, and the intervals are set to be smaller than the interval between the two first robot arms 6 and 7, so that the speed of various processes performed between the second robot arms 10 and 11 or 11 and 12 is higher. As shown in Fig. 4, an immovable fixed auxiliary arm 20 is provided at a position in the vicinity of each of the second robot arms 10, 11, and 12 and below each of the second robot arms 10, 11, and 12. The auxiliary arm 20 includes a fixed part 20A fixed to a fixing member 21, and a pair of grips 20B (in Fig. 4, only the grip 20B on the front side is shown) attached so as to be capable of moving close to and away from the fixed part 20A. In Fig. 4, for example, after the upper end part of the preparation tank 18 is held by the second robot arm 10, 11, or 12, so as to be moved to a position where it can be held by the pair of grips 20B of the auxiliary arm 20, the lower end part of the preparation tank 18 is gripped by the pair of grips 20B of the auxiliary arm 20. In this way, a cap 18A of the preparation tank 18 can be opened or closed by the single second robot arm 10, 11, or 12. Further, a program to open and close screw caps that are provided on a plurality of types of containers is stored in the second robot arms 10, 11, and 12, so that the second robot arms 10, 11, and 12 can open and close the screw caps provided on the plurality of types of containers. Therefore, it is not necessary to unify the types of containers used in the production apparatus into the same type, and thus the production apparatus of cultured cell products can be easily achieved. Also in the first robot arms 6 and 7, such a program may be stored.

Further, the two pass boxes 4 are provided to be continuou with the rear wall of the processing section 13. One (on the left side) of the pass boxes 4 is arranged so that the articles such as a plurality of types of containers including the product containers 9, the culture vessels 1, and the centrifuge tube 17, and the preparation tank 18 that is a container in which drugs are put can be carried therein passing through between the second robot arm 10 located at the left end and the second robot arm 11 located at the center. The other (on the right side) of the pass boxes 4 is arranged so that the articles are carried to the second robot arm 12 located at the right end.

As described above, the isolator 3 is horizontally elongated, in which the plurality (in this embodiment, two) of pass boxes 4 are located on the longitudinal side of the isolator 3 (in this embodiment, on the rear side). This configuration can reduce the size of the production apparatus without limiting the amount of articles to be carried into the isolator 3.

The opening of the outlet 14 is configured to have a size such that the second robot arm 12 located at the right end can easily enter therethrough, and the outlet 14 is provided with a freely openable electric shutter (not shown) and is provided continuously with the box 22 that forms a space in which the product containers 9 moved through the outlet 14 to the outside of the isolator 3 are kept for a while.

Each of the observation section 8 and the processing section 13 includes a collection container 23 made of stainless steel for collecting a culture medium, a washing liquid, and the like, which have become unnecessary. The collection container 23 includes a collection tube 23A with its diameter increasing toward the upper end side at the upper end. The collection container 23 is configured to be capable of changing its position by a guide mechanism 24 provided in each of the observation section 8 and the processing section 13 between a set position located directly below a vent formed by the opening of the collection tube 23A in each of the observation section 8 and the processing section 13 and a retracted position where the opening of the collection tube 23A is displaced from the vent so that the collection container 23 can be moved.

The processing section 13 includes: a first transfer processing unit configured to transfer a cell-containing liquid housed in the culture vessels 1 received from the first robot arm 7 into the centrifuge tube 17 using the second robot arm 10; a separation processing unit configured to separate the cells and a liquid portion by subjecting the centrifuge tube 17 to a centrifuge 26 using the second robot arm 10; and a second transfer unit configured to transfer a specified number of cells within the centrifuge tube 17 into a large number of the product containers 9 while a preservative solution (cryopreservation solution) is put into the centrifuge tube 17 after removing at least part of the liquid portion separated in the separation processing unit from the centrifuge tube 17, using the second robot arm 10. In the description of this embodiment, the term "cell-containing liquid" simply means a "liquid containing cells" and is not limited to a liquid in a specific state.

Further, the processing section 13 includes a medium-replacing unit configured to replace the culture medium within the culture vessels 1 taken out of the incubators 2 using the first robot arm 7, and the medium-replacing unit is configured to open the caps provided on the culture vessels 1 received by the second robot arm 10 from the first robot arm 7, to dispose of the culture medium within the culture vessels 1, to supply another culture medium into the cell culture vessels 1, to put the caps thereon, and to return them to the first robot arm 7.

The processing section 13 configured as above is capable of performing a first process of thawing frozen cells and seeding them, a second process (passage process) of collecting the cells and seeding them on a large number of culture vessels, and a third process of collecting the cultured cells in the culture vessels after the passage process, subdividing the collected cells, transferring them into the product containers 9, and carrying them out through the outlet 14. These processes will be described based on flowcharts. The processes described below are just examples, and there is no limitation to these examples. Therefore, it is possible to change the order of individual processes, omit or replace part of the processes, or add processes.

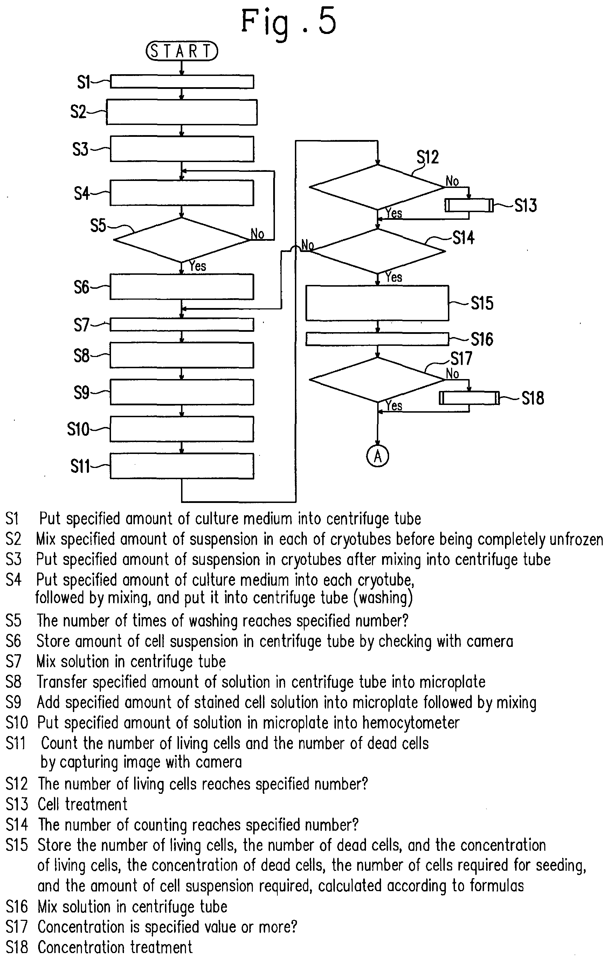

The first process will be described based on the flowcharts in Fig. 5 to Fig. 7. In these flowcharts, checking, determination, and storing are performed by the operator or the control device X, and other operations are performed by the second robot arms 10, 11, and 12. The processes not particularly mentioned are performed by the second robot arms 10, 11, and 12.

First, the centrifuge tube 17 is taken, and a specified amount of culture medium is put into the centrifuge tube 17 (S1). Next, a specified amount of suspension in each of a plurality of cryotubes (tubes for storing biological samples or the like) before being completely unfrozen is mixed (stirred) (S2), and a specified amount of suspension in all cryotubes is put together into the centrifuge tube 17 (S3). For collecting cells remaining in each cryotube, a specified amount of culture medium is put into the cryotube, followed by mixing (stirring), and is thereafter put into the centrifuge tube 17 (S4: washing). The washing is repeated a specified number of times, and the process proceeds to the next step (S5). The operator checks the amount of cell suspension within the centrifuge tube 17 by capturing an image with a camera. The operator stores the amount of liquid into the control device X (S6). After storing the amount of liquid, the solution in the centrifuge tube 17 is mixed (stirred) (S7). Subsequently, a specified amount of the solution in the centrifuge tube 17 is transferred to a microplate (S8), and a specified amount of stained cell solution is added into the microplate, followed by mixing (stirring) (S9). A specified amount of the solution in the microplate is put into a hemocytometer (S10). The operator counts the number of living cells and the number of dead cells by capturing an image with a camera (S11), checks whether the number of living cells is a specified number or more (S12), and inputs the result into the control device X if the number is less than the specified number, and the process proceeds to cell treatment (S13). If the number of living cells is the specified number or more, or after the cell treatment is performed, the control device X checks whether or not the counting has been performed a specified number of times (S14). When it is confirmed that the counting has been performed a specified number of times, the control device X stores the number of living cells, the number of dead cells, and the concentration of living cells, the concentration of dead cells, the number of cells required for seeding, and the amount of cell suspension required, which are calculated in accordance with formulas (S15). Subsequently, the solution in the centrifuge tube 17 is mixed (stirred) (S16). The operator checks whether or not the concentration is a specified value or more (S17), and the process proceeds to the next step. If the concentration does not reach the specified value or more, the process proceeds to the next step after concentration treatment is performed (S18).

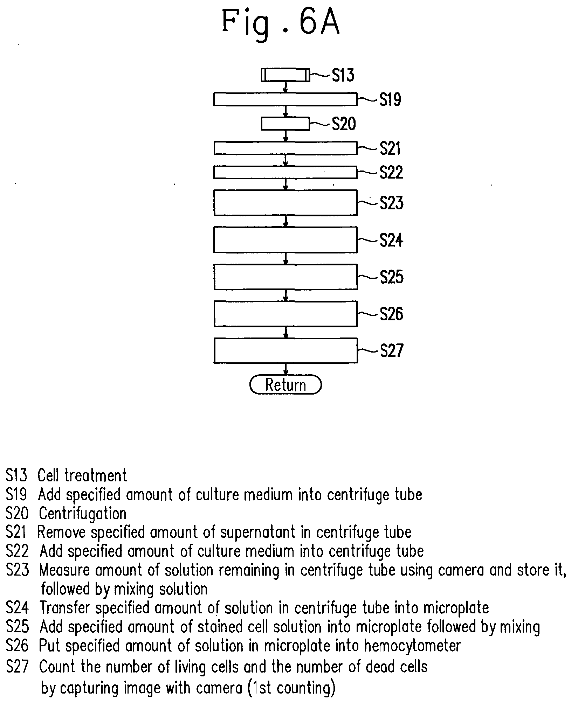

As shown in Fig. 6A, the cell treatment is performed by adding a specified amount of culture medium into the centrifuge tube 17 (S19), and putting the centrifuge tube 17 into the centrifuge 26 for centrifugation (S20). After the centrifugation, a specified amount of supernatant in the centrifuge tube 17 is removed (S21), and thereafter a specified amount of culture medium is added into the centrifuge tube 17 (S22). The operator measures the amount of solution remaining in the centrifuge tube 17 by capturing an image with a camera and stores the amount of solution into the control device X, while the second robot arm mixes (stirs) the solution (S23). A specified amount of the solution in the centrifuge tube 17 is transferred to a microplate (S24), and a specified amount of stained cell solution is added into the microplate, followed by mixing (stirring) (S25). Next, after a specified amount of the solution in the microplate is put into a hemocytometer (S26), the operator counts the number of living cells and the number of dead cells by capturing an image with a camera (S27: this is regarded as the 1st counting), and thereafter the process proceeds to step S14.

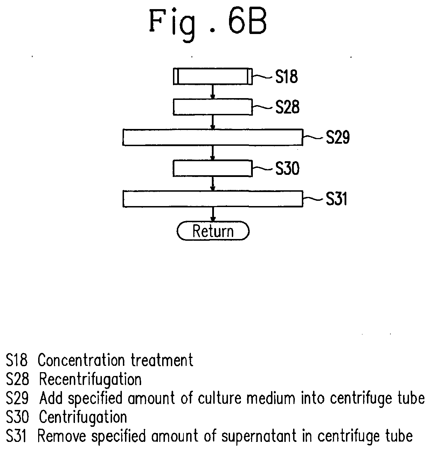

As shown in Fig. 6B, the concentration treatment is performed by putting the centrifuge tube 17 into the centrifuge 26 for recentrifugation (S28), adding a specified amount of culture medium into the centrifuge tube 17 (S29), and putting the centrifuge tube 17 into the centrifuge 26 for centrifugation (S30). After the centrifugation, a specified amount of supernatant in the centrifuge tube 17 is removed (S31), and the process proceeds to step S32.

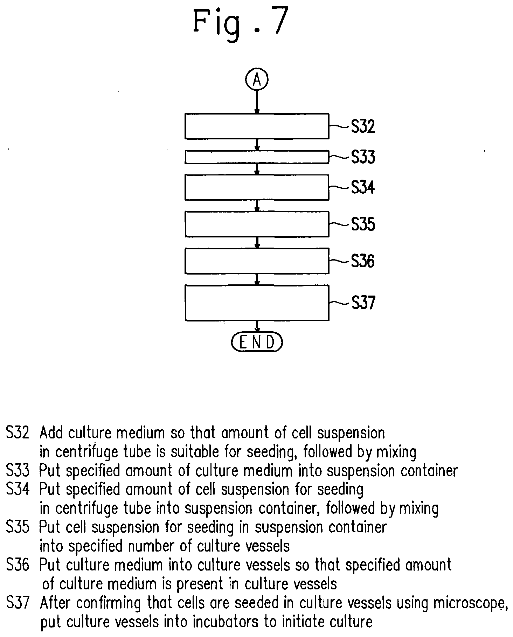

If the concentration in the centrifuge tube 17 reaches a specified concentration or more, or after the concentration treatment ends, a culture medium is added so that the amount of cell suspension in the centrifuge tube 17 is suitable for seeding, followed by mixing (stirring), as shown in Fig. 7 (S32). Next, a specified amount of culture medium is put into a suspension container (S33), and a specified amount of cell suspension for seeding (cell-containing liquid) in the centrifuge tube 17 is put into the suspension container, followed by mixing (stirring) (S34). Subsequently, the cell suspension for seeding in the suspension container is put into a specified number of the culture vessels 1 (S35), and a culture medium is put into the culture vessels 1 so that a specified amount of culture medium is present in the culture vessels 1 (S36). Thereafter, the operator confirms that the cells are seeded in the culture vessels 1 using the microscope 25 and presses the OK button provided in the production apparatus upon confirming that the cells are seeded. When the OK button is pressed, the second robot arms 10, 11, and 12 and the first robot arms 6 and 7 put the culture vessels 1 into the incubators 2 to initiate culture (S37), and the process ends.

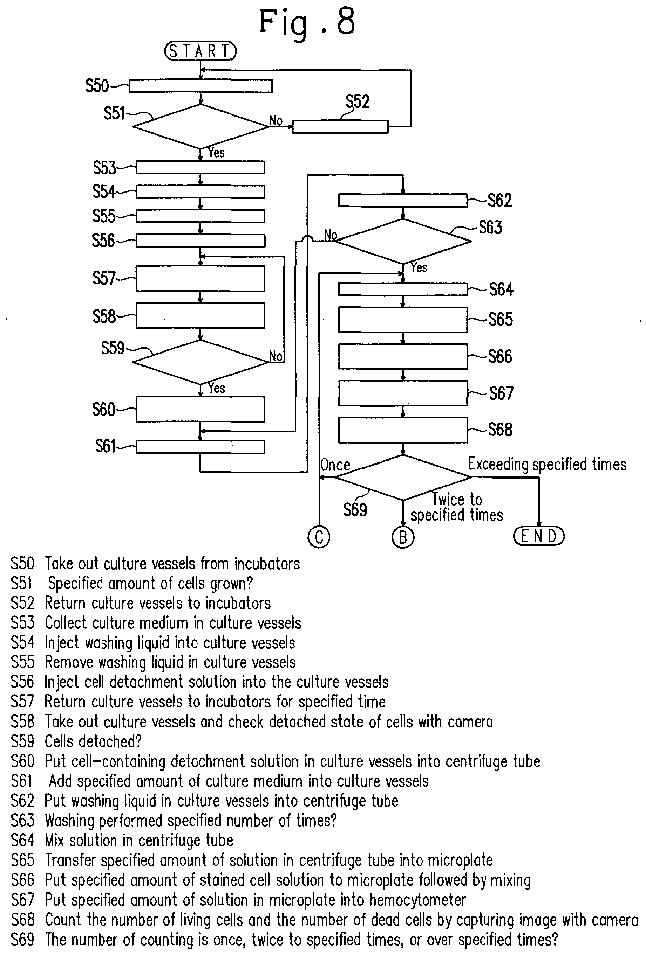

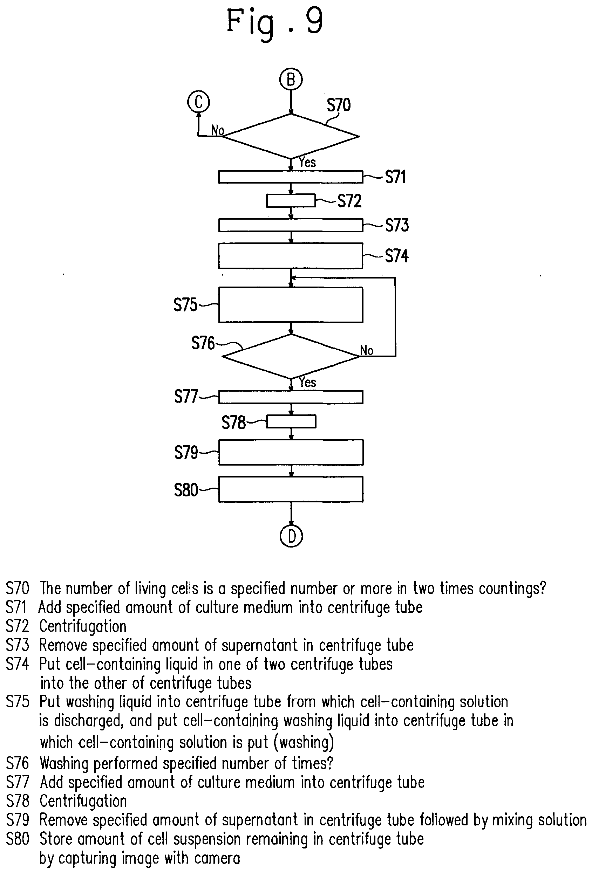

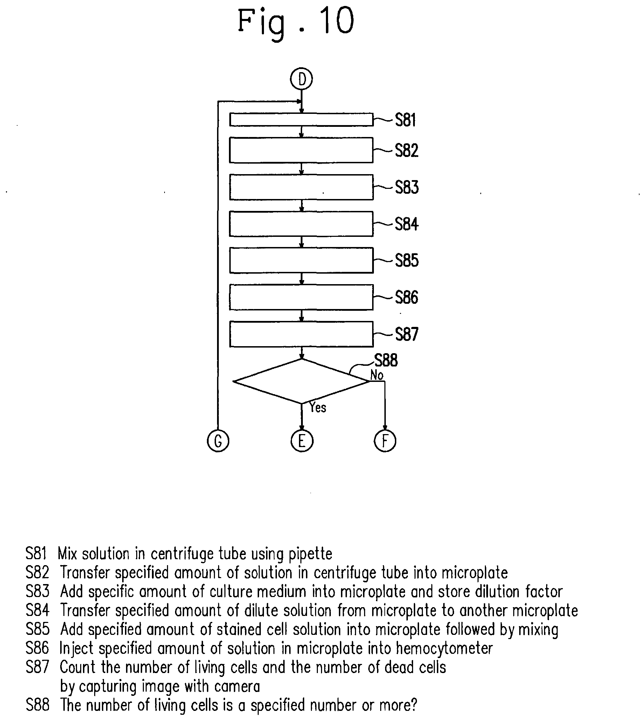

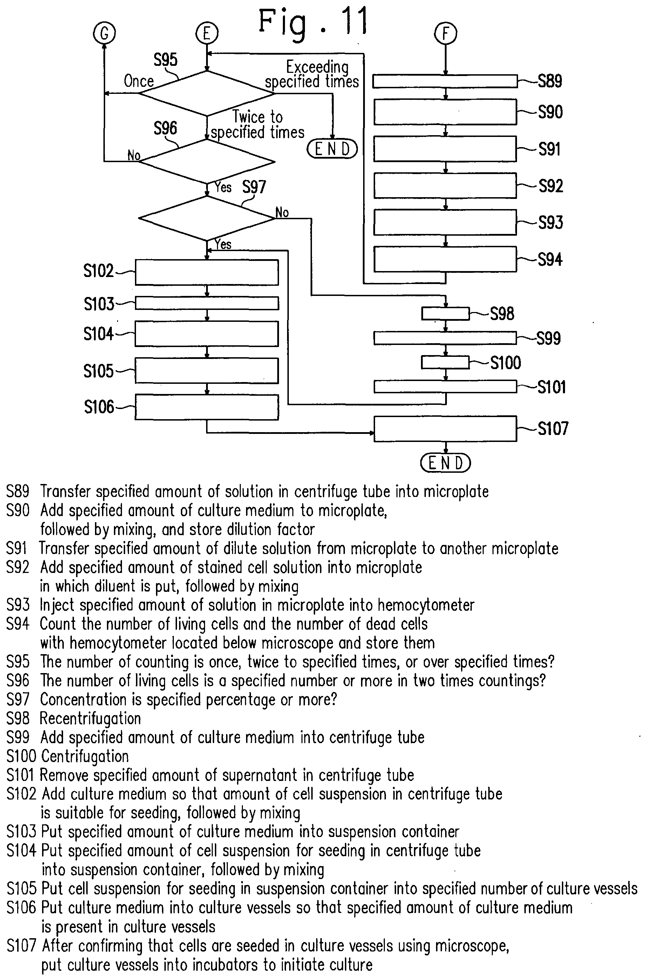

Next, the second process (passage process) will be described based on the flowcharts in Fig. 8 to Fig. 11. Also in this case, in the flowcharts, checking, determination, and storing are performed by the operator or the control device X, and other operations are performed by the second robot arms 10, 11, and 12. The processes not particularly mentioned are performed by the second robot arms 10, 11, and 12.

First, the culture vessels 1 are taken out of the incubators 2 via the first robot arms 6 and 7 (S50), it is checked whether or not a specified amount of cells has grown (S51), and if not grown, the culture vessels 1 are returned to the incubators 2 via the first robot arms 6 and 7 (S52). If grown, the culture medium in the culture vessels 1 is collected subsequently (S53), and a washing liquid (such as phosphate buffer normal saline (PBS)) is injected into the culture vessels 1 (S54). Subsequently, the washing liquid in the culture vessels 1 is removed (S55). Thereafter, a cell detachment solution (for example, a solution containing an enzyme such as trypsin) is injected into the culture vessels 1 (S56). Then, the culture vessels 1 are returned into the incubators 2 for a specified time via the first robot arms 6 and 7 (S57), the culture vessels 1 are taken out of the incubators 2 via the first robot arms 6 and 7, and the operator checks the detached state of the cells with a camera (S58). Upon determining that the cells are detached in the culture vessels 1 (S59), the operator presses the OK button provided in the production apparatus. When the OK button is pressed, the cell-containing detachment solution in the culture vessels 1 is put into the centrifuge tube 17 (S60), and a specified amount of culture medium is added into the culture vessels 1 (S61). After the addition, the washing liquid in the culture vessels 1 is put into the centrifuge tube 17 (S62). After performing the washing of step S61 and step S62 a specified number of times, the process proceeds to the next step (S63). Next, the solution in the centrifuge tube 17 is mixed (stirred) (S64), and a specified amount of the solution in the centrifuge tube 17 is transferred to a microplate (S65). Subsequently, a specified amount of stained cell solution is put into the microplate, followed by mixing (stirring) (S66), and a specified amount of the solution in the microplate is put into a hemocytometer (S67). Thereafter, the operator counts the number of living cells and the number of dead cells by capturing an image with a camera (S68). After performing the counting in step S64 to step S68 twice or more (S69), the operator checks whether or not the number of living cells is a specified number or more in two times countings (S70). Until it is confirmed twice that the number of living cells is the specified number or more, the counting is continued. If the number of counting exceeds a specified number of times, the process is ended (S69). When it is confirmed twice that the number of living cells is the specified number or more, the operator presses the OK button provided in the production apparatus. When the OK button is pressed, a specified amount of culture medium is added into the centrifuge tube 17 (S71), and the centrifuge tube 17 is put into the centrifuge 26 for centrifugation (S72). After a specified amount of supernatant in the centrifuge tube 17 is removed (S73), the cell-containing solution in one of two centrifuge tubes 17 is put into the other of two centrifuge tubes 17 (S74). A washing liquid is put into the centrifuge tube 17 from which the cell-containing solution is discharged, and the cell-containing washing liquid is put into the centrifuge tube 17 in which the cell-containing solution is put (washing) (S75). After the washing is performed a specified number of times (S76), a specified amount of culture medium is added into the centrifuge tube 17 (S77), and the centrifuge tube 17 is put into the centrifuge 26 for centrifugation (S78). After the centrifugation, a specified amount of supernatant in the centrifuge tube 17 is removed, and the solution is mixed (stirred) (S79). The operator stores the amount of the cell suspension remaining in the centrifuge tube 17 into the control device X by capturing an ᵢmage with a camera (S80). Next, the solution in the centrifuge tube 17 is mixed (stirred) using a pipette (S81), and a specified amount of solution is transferred from the inside of the centrifuge tube 17 to a microplate (S82). After the solution is transferred, a specified amount of culture medium is added into the microplate, and the dilution factor is stored (S83). After the dilution factor is stored, a specified amount of the dilute solution is transferred from the microplate to another microplate (S84), and a specified amount of stained cell solution is added into the microplate, followed by mixing (stirring) (S85). Thereafter, a specified amount of the solution in the microplate is injected into a hemocytometer (S86). The operator counts the number of living cells and the number of dead cells by capturing an image with a camera (S87). At this time, the operator checks whether or not the number of living cells is a specified number or more (S88).

If the operator determines that the number of living cells is less than the specified number, a specified amount of the solution in the centrifuge tube 17 is transferred to a microplate (S89), and a specified amount of culture medium is added into the microplate, followed by mixing (stirring), while the dilution factor is stored (S90). Next, a specified amount of the dilute solution is transferred from the microplate to another microplate (S91), and a specified amount of stained cell solution is added into the microplate in which a diluent is put, followed by mixing (stirring) (S92). Subsequently, a specified amount of solution is injected into a hemocytometer from the microplate (S93). The operator counts the number of living cells and the number of dead cells with the hemocytometer located below the microscope 25 and stores them (S94), and the process proceeds to step S95.

The operator performs the counting a specified number of times (S95) until it is determined that the number of living cells is the specified number or more at the 1st counting in step S87 ("Yes" in S88), or it is confirmed twice that the number of living cells is the specified number or more in the case where steps S89 to S94 are performed (S96). If it is not confirmed, once in step S95 or twice in step S96, that the number of living cells is the specified number or more, the process returns to step S81. If the number of counting exceeds a specified number of times, the process is ended (S95). When it is confirmed twice that the number of living cells is the specified number or more, the operator presses the OK button provided in the production apparatus. When the OK button is pressed, it is checked whether the concentration is a specified percentage or more (S97). When the concentration is not a specified percentage or more, the concentration is adjusted. That is, the centrifuge tube 17 is put into the centrifuge 26 for recentrifugation (S98). Next, a specified amount of culture medium is added into the centrifuge tube 17 (S99), and the centrifuge tube 17 is put into the centrifuge 26 for centrifugation (S100). After the centrifugation, a specified amount of supernatant in the centrifuge tube 17 is removed (S101), and the process of adjusting the concentration to a specified percentage or more is ended.

If the concentration is the specified percentage or more, or the process of adjusting the concentration to the specified percentage or more is ended, a culture medium is added so that the amount of cell suspension in the centrifuge tube 17 is suitable for seeding, followed by mixing (stirring) (S102). Next, the culture medium is put into a suspension container (S103), and a specified amount of cell suspension for seeding in the centrifuge tube 17 is put into the suspension container, followed by mixing (stirring) (S104). Subsequently, after the cell suspension for seeding in the suspension container is put into a specified number of the culture vessels 1 (S105), a culture medium is put into the culture vessels 1 so that a specified amount of culture medium is present in the culture vessels 1 (S106). Thereafter, the operator confirms that the cells are seeded in the culture vessels 1 using the microscope 25 and presses the OK button provided in the production apparatus upon the confirmation. After pressing the OK button, the operator initiates culture by putting the culture vessels 1 into the incubators 2 via the first robot arms 6 and 7 (S107), and the process is ended.

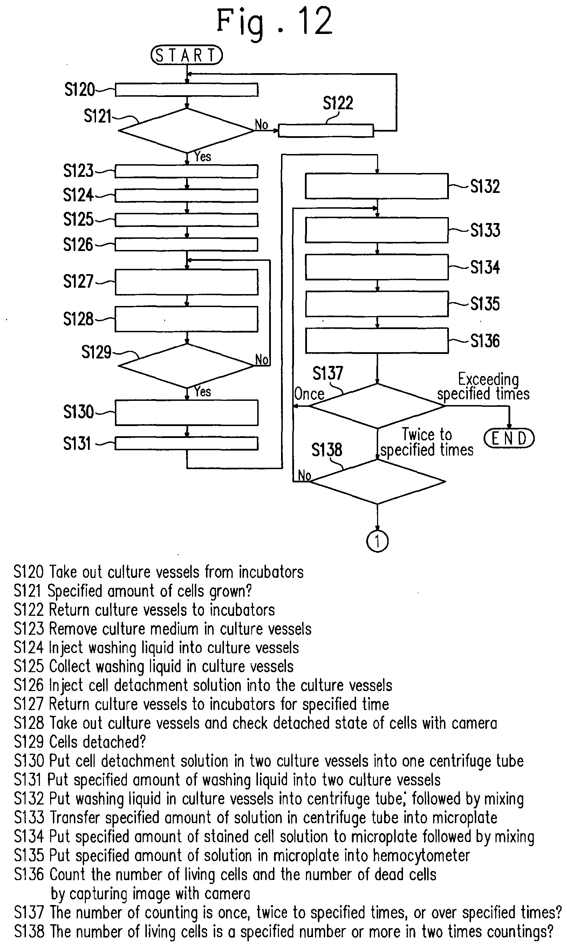

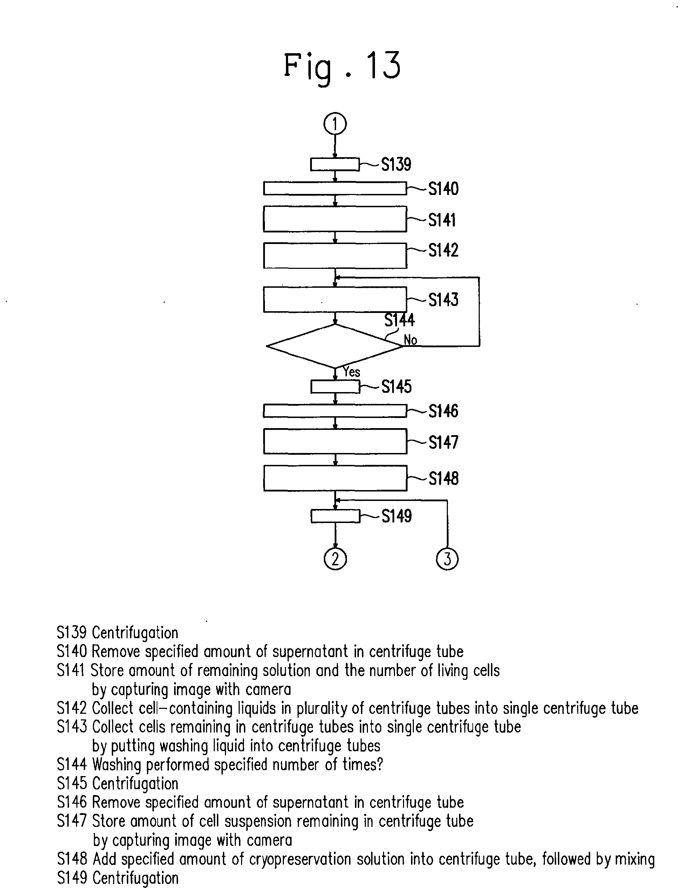

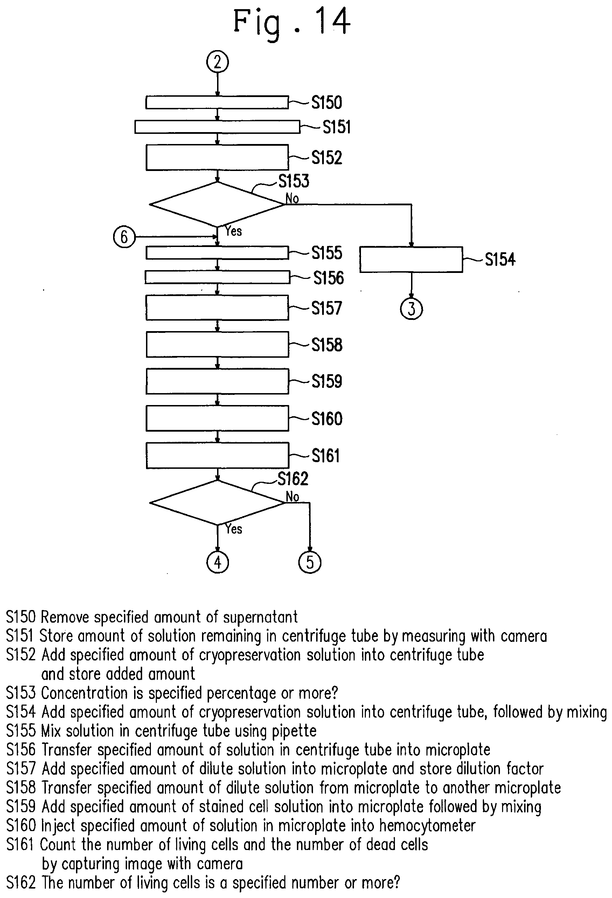

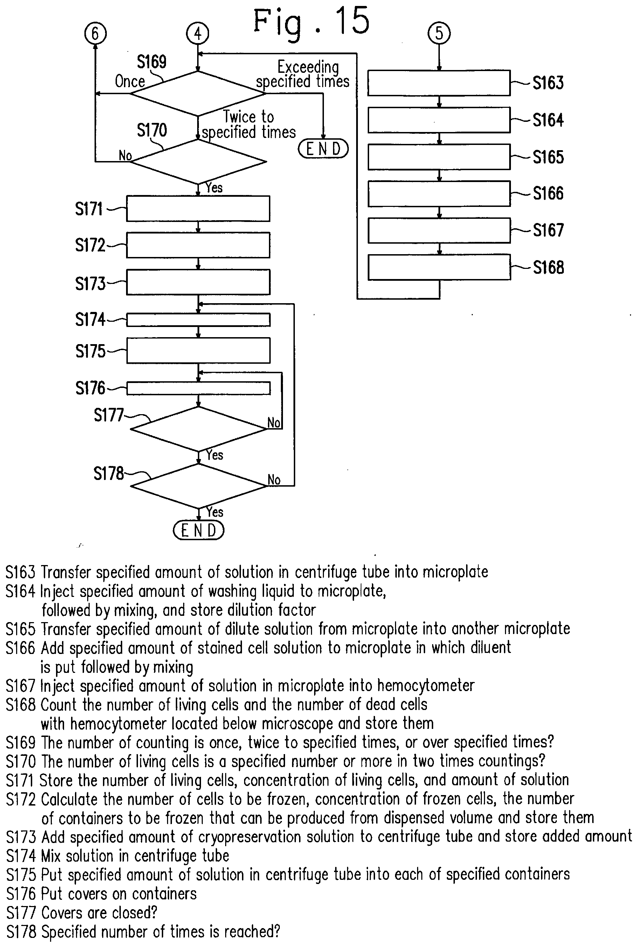

Next, the third process will be described based on the flowcharts in Fig. 12 to Fig. 15. Also in this case, in the flowcharts, checking, determination, and storing are performed by the operator or the control device X, and other operations are performed by the second robot arms 10, 11, and 12. The processes not particularly mentioned are performed by the second robot arms 10, 11, and 12.

First, the culture vessels 1 are taken out of the incubators 2 via the first robot arms 6 and 7 (S120), it is checked whether or not a specified amount of cells has grown (S121), and if not grown, the culture vessels 1 are returned to the incubators 2 via the first robot arms 6 and 7 (S122). If grown, the culture medium in the culture vessels 1 is removed subsequently (S123), and a washing liquid is injected into the culture vessels 1 (S124). Subsequently, the washing liquid in the culture vessels 1 is collected (S125). Thereafter, a cell detachment solution is injected into the culture vessels 1 (S126). Then, the culture vessels 1 are returned into the incubators 2 for a specified time via the first robot arms 6 and 7 (S127), the culture vessels 1 are taken out of the incubators 2 via the first robot arms 6 and 7, and the operator checks the detached state of the cells with a camera (S128). Upon determining that the cells are detached (S129), the operator presses the OK button provided in the production apparatus. Otherwise, the culture vessels 1 are returned again into the incubators 2 for a specified time via the first robot arms 6 and 7 (S127). When the OK button is pressed, the cell-containing detachment solutions in a plurality (herein two) of the culture vessels 1 are put (merged) into the single centrifuge tube 17 (S130), and a specified amount of washing liquid is put into the two culture vessels 1 (S131). After the addition, the washing liquid in the culture vessels 1 is put into the centrifuge tube 17, followed by mixing (stirring) (S132). Next, a specified amount of the solution in the centrifuge tube 17 is transferred to a microplate (S133). Subsequently, a specified amount of stained cell solution is put into the microplate, followed by mixing (stirring) (S134), and a specified amount of the solution in the microplate is put into a hemocytometer (S135). Thereafter, the operator counts the number of living cells and the number of dead cells by capturing an image with a camera (S136). After performing the counting in step S133 to step S136 twice or more (S137), the operator checks whether or not the number of living cells is a specified number or more in two times countings (S138). This checking is performed for checking the total number of cells that can be produced. It is better to perform the checking, but it is also possible to perform only the subsequent step S162 by omitting the checking. The counting is continued until it is confirmed twice that the number of living cells is the specified number or more. If the number of counting exceeds a specified number of times, the process is ended (S137). When it is confirmed twice that the number of living cells is the specified number or more, the operator presses the OK button provided in the production apparatus. When the OK button is pressed, the centrifuge tube 17 is put into the centrifuge 26 for centrifugation (S139). After a specified amount of supernatant in the centrifuge tube 17 is removed (S140), the operator captures an image to measure the amount of solution and the number of living cells in the centrifuge tube 17 with a camera. The operator stores the amount of solution and the number of living cells into the control device X (S141). Next, the cell-containing liquids in a plurality of centrifuge tubes 17 are collected (merged) into a single centrifuge tube 17 (S142). At this time, in order to collect, into the single centrifuge tube 17 into which the cell-containing liquids are collected, the cells remaining in the centrifuge tubes 17 except for the single centrifuge tube 17, a washing liquid is put into each of the centrifuge tubes 17, and the cell-containing washing liquids are put into the single centrifuge tube 17 (washing) (S143). After the washing is performed a specified number of times (S144), and the centrifuge tube 17 is put into the centrifuge 26 for centrifugation (S145). After the centrifugation, a specified amount of supernatant in the centrifuge tube 17 is removed (S146), and the operator stores the amount of the cell suspension remaining in the centrifuge tube 17 into the control device X by capturing an image with a camera (S147). Next, a specified amount of cryopreservation solution is added into the centrifuge tube 17, followed by mixing (stirring) (S148), and the centrifuge tube 17 is put into the centrifuge 26 for centrifugation (S149). After the centrifugation, a specified amount of supernatant in the centrifuge tube 17 is removed (S150), and the operator measures the amount of solution remaining in the centrifuge tube 17 by capturing an image with a camera and stores it in the control device X (S151). Subsequently, a specified amount of cryopreservation solution is added into the centrifuge tube 17, and the operator stores the added amount in the control device X (S152).

Next, the operator checks whether or not the concentration of the solvent (such as DMSO (dimethylsulfoxide)) in the preservative solution within the centrifuge tube 17 is a specified percentage or more (S153). If the concentration in the centrifuge tube 17 is not the specified percentage or more, the concentration is adjusted. That is, a specified amount of cryopreservation solution is added into the centrifuge tube 17, followed by mixing (stirring) (S154), and after the process of step S149 to step S152 is performed, the process proceeds to the next step. If the concentration in the centrifuge tube 17 is the specified percentage or more, or after a specified amount of cryopreservation solution is added into the centrifuge tube 17, followed by mixing (stirring) (S154), and the process of step S149 to step S152 is performed, the solution in the centrifuge tube 17 is mixed (stirred) using a pipette (S155), and a specified amount of solution is transferred from the inside of the centrifuge tube 17 to a microplate (S156). After the solution is transferred, a specified amount of dilute solution is added into the microplate, and the dilution factor is stored (S157). After the dilution factor is stored, a specified amount of the dilute solution is transferred from the microplate to another microplate (S158), and a specified amount of stained cell solution (such as trypan blue) is added into the microplate, followed by mixing (stirring) (S159). Thereafter, the specified amount in the microplate is injected into a hemocytometer (S160). The operator counts the number of living cells and the number of dead cells by capturing an image with a camera (S161). At this time, the operator checks whether or not the number of living cells is a specified number or more (S162). This checking is performed for checking the final number of living cells and the concentration thereof, in order to define the necessary amount of liquid when filling final product containers, since some cells may die during the process in some cases.

If the operator determines that the number of living cells is less than the specified number, a specified amount of the solution in the centrifuge tube 17 is transferred to a microplate (S163), a specified amount of washing liquid is injected into the microplate, followed by mixing (stirring), and the dilution factor is stored (S164). Next, a specified amount of the dilute solution is transferred from the microplate to another microplate (S165), and a specified amount of stained cell solution is added into the microplate into which a diluent is put, followed by mixing (stirring) (S166). Subsequently, a specified amount of solution is injected into a hemocytometer from the microplate (S167). The operator counts the number of living cells and the number of dead cells with the hemocytometer located below the microscope 25 and stores them (S168), and the process proceeds to step S169.

The operator performs the counting a specified number of times (S169) until it is determined that the number of living cells is the specified number or more at the 1st counting in step S161 ("Yes" in S162), or it is confirmed twice that the number of living cells is the specified number or more in the case where steps S163 to S168 are performed (S170). If it is not confirmed, once in step S169 or twice in step S170, that the number of living cells is the specified number or more, the process returns to step S155. If the number of counting exceeds a specified number of times, the process is ended (S169). When it is confirmed twice that the number of living cells is the specified number or more, the operator presses the OK button provided in the production apparatus. When the OK button is pressed, the operator stores the number of living cells, the concentration of living cells, and the amount of liquid in the control device X (S171). Subsequently, the operator calculates the number of cells to be frozen, the concentration of frozen cells, the number of containers (the product containers 9) to be frozen that can be produced from the dispensed volume and stores them in the control device X (S172). Then, the operator adds a cryopreservation solution to the centrifuge tube 17 in an amount such that the defined concentration of frozen cells as final cultured cell products is achieved and stores the addition amount in the control device X (S173). Next, the solution in the centrifuge tube 17 is mixed (stirred) (S174), and a specified amount of the solution in the centrifuge tube 17 is put into each of the containers (the product containers 9) (S175). After the completion of the input, caps are put on the containers (the product containers 9) (S176), and the operator checks whether or not the caps are closed using a camera (S177). At this time, the caps are still temporarily fixed. If it is not confirmed that the caps are closed in step S177, the process returns to step S176. When the control device X determines that the operation to put the solution into the containers (the product containers 9) is performed a specified number of times (S178), the process is ended. The containers (the product containers 9) after the input are moved through the outlet 14 into the box 22. If the control device X does not determine in step S178 that the operation to put the solution into the containers (the product containers 9) is performed a specified number of times, the process returns to step S174.

Though not shown in the flowcharts, the containers (the product containers 9) moved into the box 22 are taken out of the box 22, the caps are crimped, and labels are pasted to accomplish the cultured cell products, by the operator. The cultured cell products are frozen and circulate in the frozen state (which are delivered to medical institution or research institution).

In the aforementioned third process, the production apparatus performs a taking-out step (which corresponds to steps S120 to S147 described in the flowcharts) of taking out the cells cultured in the culture vessels 1, a cell density-adjusting step (which corresponds to steps S149 to S171 therein) of adjusting the density of the cells in a cell-containing liquid containing the taken-out cells, and a subdividing step (which corresponds to S175 to S178 therein) of subdividing the cell-containing liquid with its density adjusted and putting it into the plurality of product containers 9, to accomplish the cultured cell products. The relationship between these steps and the respective processes described in the flowcharts is just an example, and there is no limitation to the aforementioned correlation (the same applies to the following correlation).

By performing the cell density-adjusting step, the quality of the produced cultured cell products can be made uniform, and therefore the produced cultured cell products have high quality (specifically, the number of living cells in the cultured cell products is made uniform).

Further, the cell density-adjusting step includes a counting support step (which corresponds to steps S155 to S161 and S163 to S168 described in the flowcharts) in which the production apparatus supports the counting of the number of living cells by observing partially or entirely the plurality of cells taken out by the taking-out step, and a preservative solution-adding step (which corresponds to step S173 therein) of adding a preservative solution (cryopreservation solution) to the taken-out cells in an amount corresponding to the number of living cells obtained by the counting. By performing the counting support step, the counting performed by the operator or the like can be performed rapidly by being supported by the production apparatus. Then, the amount of preservative solution is determined depending on the number of living cells obtained by performing the counting support step. Therefore, a liquid adjusted to have a specified density of living cells is rapidly obtained.

Further, prior to performing the subdividing step, the production apparatus performs a container number-deriving step (which corresponds to step S172 described in the flowchart) of deriving the number of the product containers 9 based on the number of living cells obtained by the counting. In this step, the number of the product containers 9 to be prepared is determined before the subdividing step, and therefore the subdividing and placing into the product containers 9 can be rapidly performed. In this embodiment, the number of the product containers 9 is derived by calculation, but it may be derived without calculation.

Further, before the cell density-adjusting step and the subdividing step, suspension steps (which corresponds to steps S148 and S174 described in the flowcharts) are performed. Such a suspension step is a step of forming a suspension in which the cells are uniformly dispersed by stirring the cell-containing liquid. In the suspension after the suspension steps, the cultured cells are uniformly dispersed, and therefore the density of living cells to be taken out in the cell density-adjusting step and the subdividing step can be made uniform. This can contribute to making the quality of the cultured cell products uniform.

The apparatus to produce cultured cell products according to the present invention is not limited to the aforementioned embodiment, and various modifications can be made without departing from the gist of the present invention.

In the embodiment, the two robot arms 6 and 7 are provided in the observation section 8, and the three robot arms 10, 11, and 12 are provided in the processing section 13, but the implementation is also possible by providing at least one robot arm in the observation section 8 and providing at least one robot arm in the processing section 13.

Further, in the embodiment, the isolator 3 is configured to have a horizontally elongated shape but may be configured to have a square shape or a circular shape. Further, it may be configured to have a bent shape.

Further, in the embodiment, the microscopes 16 and 25 are used for observing the cells, but there is no limitation to the microscopes, and it is possible to use various image enlarging devices that can enlarge captured images of cells to be observed. The number of living cells can be accurately counted by using such an image enlarging device.

The configuration and action of the aforementioned embodiment will be summarized below. The apparatus to produce cultured cell products according to the embodiment includes: an incubator 2 configured to house cell culture vessels 1; an isolator 3 capable of maintaining its inside in aseptic conditions and configured to process the cell culture vessels 1 conveyed from the incubator 2; and a pass box 4 capable of carrying, into the isolator 3, articles such as a plurality of types of containers including product containers 9 and reagents which are used for subdividing and placing the cells cultured in the cell culture vessels 1, wherein the isolator 3 includes: an observation section 8 including one or a plurality of first robot arms 6 and 7 configured to move the cell culture vessels 1 to an observation position so as to check the degree of growth of the cells in the cell culture vessels 1 taken out of the incubator 2; a processing section 13 provided continuously with the observation section 8 and including one or a plurality of second robot arms 10 to 12 configured to perform various processes to transfer the cells in the cell culture vessels 1 that have a specified number of cells out of the cell culture vessels 1 observed in the observation section 8 into a large number of the product containers 9 carried in from the pass box 4; and an outlet 14 through which the large number of product containers 9 into which the cells have been transferred are taken out.

According to such a configuration, the observation section 8 and the processing section 13 are separated from each other, and therefore, even while the cells in the cell culture vessels 1 moved to the observation position by the first robot arms 6 and 7 are being observed, various processes to transfer the cells in the cell culture vessels 1 moved from the observation section 8 to the processing section 13 using the second robot arms 10 to 12 into a large number of the product containers 9 can be performed, thereby enabling rapid processing. This allows a large amount of cells to be cultured and the large amount of cultured cells to be subdivided and placed into a large number of the product containers 9, which can be taken out through the outlet 14.

Further, in the apparatus to produce cultured cell products according to the embodiment, the processing section 13 may include: a first transfer processing unit configured to transfer a cell-containing liquid housed in the cell culture vessels 1 received from the first robot arms 6 and 7 into a centrifuge tube 17 using the second robot arms 10 to 12; a separation processing unit configured to separate the cells and a liquid portion by subjecting the centrifuge tube 17 to a centrifuge 26 using the second robot arms 10 to 12; and a second transfer unit configured to at least partially remove the liquid portion separated by the separation processing unit from the centrifuge tube 17, put a preservative solution into the centrifuge tube, and transfer a specified number of cells within the centrifuge tube into the large number of product containers while maintaining such a state, using the second robot arms 10 to 12.

According to such a configuration, the processing section 13 includes the first transfer processing unit, the separation processing unit, and the second transfer unit, and therefore, after the second robot arms 10 to 12 transfer the cell-containing liquid from the cell culture vessels 1 to the centrifuge tube 17, they can transfer a specified number of cells into the large number of product containers 9.

Further, in the apparatus to produce cultured cell products according to the embodiment, the processing section 13 includes a medium-replacing unit configured to replace a culture medium in the cell culture vessels 1 taken out of the incubator 2 using the second robot arms 10 to 12, wherein the medium-replacing unit may be configured to open the caps provided on the cell culture vessels 1 received from the first robot arms 6 and 7, so as to dispose of the culture medium in the cell culture vessels 1, supply another culture medium into the cell culture vessels 1, and put the caps on the cell culture vessels 1 to return the cell culture vessels 1 to the first robot arms 6 and 7, using the second robot arms 10 to 12.

According to such a configuration, even while the cells in the cell culture vessels 1 moved to the observation position by the first robot arms 6 and 7 are being observed, the process to replace the medium in the cell culture vessels 1 moved from the observation section 8 to the processing section 13 can be performed using the second robot arms 10 to 12.

Further, in the apparatus to produce cultured cell products according to the embodiment, the second robot arms 10 to 12 may store a program for opening and closing the screw caps provided on the plurality of types of containers.

According to such a configuration, the second robot arms 10 to 12 can open and close the screw caps provided on the plurality of types of containers, and therefore it is not necessary to use the same type of containers, so that the apparatus to produce cultured cell products can be easily achieved.

As described above, in the embodiment, the observation section 8 and the processing section 13 are separated from each other, so that the processing speed can be increased, and therefore it is possible to provide an apparatus to produce cultured cell products which can produce a large amount of cultured cell products by culturing a large amount of cells and can subdivide the large amount of cultured cells.

## Claims

1. An apparatus to produce cultured cell products, comprising:
an incubator (2) configured to house cell culture vessels (1);
an isolator (3) capable of maintaining its inside in aseptic conditions and configured to process the cell culture vessels conveyed from the incubator; and
a pass box (4) capable of carrying, into the isolator, articles such as a plurality of types of containers including product containers and reagents which are used for subdividing and placing the cells cultured in the cell culture vessels, wherein
the isolator comprises:
an observation section (8) comprising first robot arms (6, 7) configured to move the cell culture vessels to an observation position so that the degree of growth of the cells in the cell culture vessels taken out of the incubator is checked;
a processing section (13) provided continuously with the observation section and comprising second robot arms (10, 11, 12) configured to perform various processes to transfer the cells as a cell-containing liquid in the cell culture vessels that have a specified number of cells out of the cell culture vessels observed in the observation section into a large number of product containers carried in from the pass box; and
an outlet (14) configured to allow the large number of product containers into which the cells have been transferred to be taken out from the isolator to the outside of the apparatus, wherein
at least one first robot arm corresponds to the incubator and is configured to handle the culture vessels that are housed in the incubator,
at least one second robot arm corresponds to the pass box and is configured to handle the articles and the reagents to be housed or that have been housed in the pass box,
at least one second robot arm corresponds to the outlet and is configured to handle the product containers to be moved from the outlet to the outside of the isolator, and
a first robot arm can directly deliver the articles to a second robot arm.

2. The apparatus to produce cultured cell products according to claim 1, wherein
the processing section comprises:
a first transfer processing unit configured to transfer a cell-containing liquid housed in the cell culture vessels received from at least one first robot arm into a centrifuge tube, using at least one second robot arm;
a separation processing unit configured to separate the cells and a liquid portion by subjecting the centrifuge tube to centrifugation using at least one second robot arm; and
a second transfer unit configured to at least partially remove the liquid portion separated by the separation processing unit from the centrifuge tube, put a preservative solution into the centrifuge tube, and transfer a specified number of cells within the centrifuge tube into the large number of product containers while maintaining such a state, using at least one second robot arm.

3. The apparatus to produce cultured cell products according to claim 1 or 2, wherein
the processing section comprises a medium-replacing unit configured to replace a culture medium in the cell culture vessels taken out of the incubator using at least one second robot arm, wherein
the medium-replacing unit is configured to open caps provided on the cell culture vessels received from at least one first robot arm to dispose of the culture medium in the cell culture vessels, supply another culture medium into the cell culture vessels, and put the caps on the cell culture vessels to return the cell culture vessels to at least one first robot arm, using at least one second robot arm.

4. The apparatus to produce cultured cell products according to any one of claims 1 to 3, wherein
at least one second robot arm is configured to open and close screw caps provided on the plurality of types of containers.

## Patentansprüche

1. Vorrichtung zur Herstellung von gezüchteten Zellprodukten, aufweisend:
einen Inkubator (2), der dafür ausgelegt ist, Zellkulturgefäße (1) aufzunehmen;
einen Isolator (3), der in der Lage ist, in seinem Inneren aseptische Bedingungen aufrechtzuerhalten, und dafür ausgelegt ist, die Zellkulturgefäße zu bearbeiten, die vom Inkubator kommend überbracht werden; und
eine Durchgangsbox (4), die in der Lage ist, in den Isolator Gegenstände wie zum Beispiel eine Vielzahl von Typen von Behältern einzuführen, darunter Produktbehälter und Reagenzien, die zum Unterteilen und Platzieren der in den Zellkulturgefäßen gezüchteten Zellen verwendet werden, wobei
der Isolator Folgendes aufweist:
einen Beobachtungsabschnitt (8) mit ersten Roboterarmen (6, 7), die dafür ausgelegt sind, die Zellkulturgefäße zu einer Beobachtungsposition zu bewegen, so dass der Wachstumsgrad der Zellen in den dem Inkubator entnommenen Zellkulturgefäßen überprüft wird;
einen Verarbeitungsabschnitt (13), der durchgehend mit dem Beobachtungsabschnitt vorgesehen ist und zweite Roboterarme (10, 11, 12) aufweist, die dafür ausgelegt sind, verschiedene Prozesse vorzunehmen, um die Zellen als zellhaltige Flüssigkeit in den Zellkulturgefäßen, die über eine spezifizierte Anzahl von Zellen verfügen, aus den Zellkulturgefäßen, die im Beobachtungsabschnitt beobachtet werden, in eine große Anzahl von Produktbehältern zu transferieren, die von der Durchgangsbox her eingeführt werden; und
einen Auslass (14), der dafür ausgelegt ist, zu ermöglichen, die große Anzahl von Produktbehältern, in welche die Zellen transferiert wurden, aus dem Isolator zur Außenseite der Vorrichtung hin zu entnehmen, wobei
zumindest ein erster Roboterarm mit dem Inkubator korrespondiert und dafür ausgelegt ist, die Kulturgefäße zu handhaben, die im Inkubator aufgenommen sind,
zumindest ein zweiter Roboterarm mit der Durchgangsbox korrespondiert und dafür ausgelegt ist, die Gegenstände und die Reagenzien zu handhaben, die in der Durchgangsbox aufzunehmen sind oder dort aufgenommen wurden,
zumindest ein zweiter Roboterarm mit dem Auslass korrespondiert und dafür ausgelegt ist, die Produktbehälter zu handhaben, die vom Auslass zur Außenseite des Isolators zu bewegen sind, und
ein erster Roboterarm die Gegenstände direkt einem zweiten Roboterarm übergeben kann.

2. Vorrichtung zur Herstellung von gezüchteten Zellprodukten nach Anspruch 1, wobei
der Verarbeitungsabschnitt Folgendes aufweist:
eine erste Transferverarbeitungseinheit, die dafür ausgelegt ist, eine zellhaltige Flüssigkeit, die in den Zellkulturgefäßen aufgenommen ist, welche von zumindest einem ersten Roboterarm empfangen werden, unter Verwendung von zumindest einem zweiten Roboterarm in ein Zentrifugenröhrchen zu transferieren;
eine Trennverarbeitungseinheit, die dafür ausgelegt ist, die Zellen und einen Flüssigkeitsanteil zu trennen, indem das Zentrifugenröhrchen unter Verwendung von zumindest einem zweiten Roboterarm einer Zentrifugierung unterzogen wird; und
eine zweite Transfereinheit, die dafür ausgelegt ist, den durch die Trennverarbeitungseinheit abgetrennten Flüssigkeitsanteil zumindest teilweise aus dem Zentrifugenröhrchen zu entfernen, eine Konservierungslösung in das Zentrifugenröhrchen zu geben und eine spezifizierte Anzahl von Zellen in dem Zentrifugenröhrchen unter Beibehaltung eines solchen Zustands in die große Anzahl von Produktbehältern zu transferieren, und zwar unter Verwendung von zumindest einem zweiten Roboterarm.

3. Vorrichtung zur Herstellung von gezüchteten Zellprodukten nach Anspruch 1 oder 2, wobei
der Verarbeitungsabschnitt eine Medium-Ersetzungseinheit aufweist, die dafür ausgelegt ist, ein Kulturmedium in den dem Inkubator entnommenen Zellkulturgefäßen unter Verwendung von zumindest einem zweiten Roboterarm zu ersetzen, wobei
die Medium-Ersetzungseinheit dafür ausgelegt ist, Kappen zu öffnen, die an den Zellkulturgefäßen vorgesehen sind, welche von zumindest einem ersten Roboterarm erhalten werden, um das Kulturmedium in den Zellkulturgefäßen zu entsorgen, ein anderes Kulturmedium in die Zellkulturgefäße einzubringen und die Kappen auf die Zellkulturgefäße aufzusetzen, um die Zellkulturgefäße zu zumindest einem ersten Roboterarm unter Verwendung von zumindest einem zweiten Roboterarm zurückzubringen.

4. Vorrichtung zur Herstellung von gezüchteten Zellprodukten nach einem der Ansprüche 1 bis 3, wobei
zumindest ein zweiter Roboterarm dafür ausgelegt ist, Schraubkappen zu öffnen und zu schließen, die an der Vielzahl von Typen von Behältern vorgesehen sind.

## Revendications

1. Appareil destiné à produire des produits de cellules après culture, comprenant :
un incubateur (2) configuré pour loger des récipients de culture cellulaire (1) ;
un isolateur (3) capable de maintenir son intérieur dans des conditions aseptiques et configuré pour traiter les récipients de culture cellulaire acheminés à partir de l'incubateur ; et
une boîte de passage (4) capable de transporter, dans l'isolateur, des articles tels qu'une pluralité de types de contenants incluant des contenants de produits et de réactifs qui sont utilisés pour subdiviser et placer les cellules cultivées dans les récipients de culture cellulaire, dans lequel
l'isolateur comprend :
une section d'observation (8) comprenant des premiers bras robotisés (6, 7) configurés pour déplacer les récipients de culture cellulaire vers une position d'observation de manière à vérifier le degré de croissance des cellules dans les récipients de culture cellulaire retirés de l'incubateur ;
une section de traitement (13) fournie de manière continue avec la section d'observation et comprenant des deuxièmes bras robotisés (10, 11, 12) configurés pour réaliser divers processus pour transférer les cellules sous forme de liquide contenant des cellules dans les récipients de culture cellulaire qui ont un nombre spécifié de cellules en dehors des récipients de culture cellulaire observés dans la section d'observation jusque dans un grand nombre de contenants de produits transportés à partir de la boîte de passage ; et
une sortie (14) configurée pour permettre de retirer le grand nombre de contenants de produits dans lesquels les cellules ont été transférées à partir de l'isolateur vers l'extérieur de l'appareil, dans lequel
au moins un premier bras robotisé correspond à l'incubateur et est configuré pour manipuler les récipients de culture qui sont logés dans l'incubateur,
au moins un deuxième bras robotisé correspond à la boîte de passage et est configuré pour manipuler les articles et les réactifs à loger ou qui ont été logés dans la boîte de passage,
au moins un deuxième bras robotisé correspond à la sortie et est configuré pour manipuler les contenants de produits à déplacer à partir de la sortie vers l'extérieur de l'isolateur, et
un premier bras robotisé peut délivrer directement les articles vers un deuxième bras robotisé.

2. Appareil de production de produits de cellules après culture selon la revendication 1, dans lequel
la section de traitement comprend :
une première unité de traitement de transfert configurée pour transférer un liquide contenant des cellules logé dans les récipients de culture cellulaire reçus à partir d'au moins un premier bras robotisé jusque dans un tube à centrifuger, à l'aide d'au moins un deuxième bras robotisé ;
une unité de traitement de séparation configurée pour séparer les cellules et une portion de liquide en soumettant le tube à centrifuger à une centrifugation à l'aide d'au moins un deuxième bras robotisé ; et
une seconde unité de transfert configurée pour enlever au moins partiellement la portion de liquide séparée par l'unité de traitement de séparation à partir du tube à centrifuger, placer une solution de conservation dans le tube à centrifuger et transférer un nombre spécifié de cellules à l'intérieur du tube à centrifuger dans le grand nombre de contenants de produits tout en maintenant un tel état, à l'aide d'au moins un deuxième bras robotisé.

3. Appareil de production de produits de cellules après culture selon la revendication 1 ou 2, dans lequel
la section de traitement comprend une unité de remplacement de milieu configurée pour remplacer un milieu de culture dans les récipients de culture cellulaire retirés de l'incubateur à l'aide d'au moins un deuxième bras robotisé, dans lequel
l'unité de remplacement de milieu est configurée pour ouvrir des capuchons fournis sur les récipients de culture cellulaire reçus à partir d'au moins un premier bras robotisé afin de disposer le milieu de culture dans les récipients de culture cellulaire, apporter un autre milieu de culture dans les récipients de culture cellulaire et placer les capuchons sur les récipients de culture cellulaire afin de retourner les récipients de culture cellulaire vers au moins un premier bras robotisé, à l'aide d'au moins un deuxième bras robotisé.

4. Appareil de production de produits de cellules après culture selon l'une quelconque des revendications 1 à 3, dans lequel
au moins un deuxième bras robotisé est configuré pour ouvrir et fermer des capuchons vissés fournis sur la pluralité de types de contenants.
